# EUROPEAN PATENT APPLICATION

(11) **EP 4 674 373 A1**
(43) Date of publication of application: **07.01.2026**
(21) Application number: 25182641.8
(22) Date of filing: 13.06.2025
(51) Int. Cl.: A61B 34/37, A61B 90/13, A61B 90/30, A61F 9/00

(54) **LASER-GUIDED SUBRETINAL INJECTION AID**

(30) Priority: 03.07.2024 DE 102024118840
(71) Applicant: Carl Zeiss Meditec AG, 07745 Jena (DE)
(72) Inventor: Buelow, Yorck, 73447 Oberkochen (DE); Klusmann, Carolin, 73447 Oberkochen (DE); Kienle, Patrick, 81379 München (DE)
(74) Representative: Carl Zeiss AG - Patentabteilung

(57) **Abstract**

Disclosed herein are system, apparatus, article of manufacture, method, computer program product embodiments, and combinations and sub-combinations thereof, for aiding an injection in an intraocular procedure including a robot arm, a control unit configured to control the robot arm, a targeting instrument mounted on the robot arm, wherein the targeting instrument is configured to aim at a targeted position, wherein an orientation of the robot arm in a direction of the targeted position is saved in a memory of the control unit to be accessed, and wherein the targeting instrument is further configured to be exchanged with an injection instrument, and the injection instrument mounted on the robot arm, wherein the injection instrument is configured to be positioned at the saved orientation of the robot arm at the targeted position, and wherein the injection instrument is further configured to inject into the targeted position.

## Description

### TECHNICAL FIELD

The present disclosure is generally directed to a surgical instrument mounted on a surgical robot arm to assist and guide an injection for minimally invasive intraocular surgery.

### BACKGROUND

Surgical robots have been performing minimally invasive surgeries in recent years, including intraocular surgeries. Robot assisted surgeries may enhance the capabilities of surgeons that are performing surgery. Often surgeons may use a computer with a controller to control surgical instruments used during the surgery, as opposed to using their own hands. The controller allows the surgeon to perform movements associated with the surgery, but the arms of the robot make the movements via instruction from the controller using a variety of different surgical instruments, depending on the surgery. Similarly, a computer-controlled system may control the robotic arms after inputs from the surgeon.

Abnormalities or diseases of the retina are typically treated by various types of surgeries such as vitrectomy or subretinal injections, using such robots. The surgery may remove substances that keep light from focusing properly on the retina or remove scar tissue that is wrinkled or tearing the retina, which causes poor vision. Additionally, such surgeries may fix retinal detachment or repair macular holes. Within the eye, buildup of coagulated protein chains makes it difficult to move laterally inside the eye with certain surgical instruments. The movement within the eye is difficult as the vitreous material is connected to the retinal tissue and such lateral movements within the vitreous material may lead to tearing of the retina, which is undesirable. To treat different points of interest along the retina and minimize traction on the retina, moving the surgical instrument to the position of interest in a straight trajectory from a trocar is desirable.

Typically, subretinal injections require vitrectomy, an eye surgery step capable of treating various problems with the retina and vitreous and must be performed on patients before the actual subretinal injection. Vitrectomy requires a change in trajectory of a vitrectome or vitreous cutter during the approach to the retina. In case the vitrectome unintendedly collides with the posterior surface of the lens, this may lead to cataracts forming in the patient eyes, which then requires further surgeries and procedures. Typically, the fluid in the eye will be removed, or sucked out, and replaced by water, silicon oil, or saline in vitrectomy, however, this bears a lot of risks and possible problems afterwards.

Accordingly, a system for moving surgical instruments in a direct line, or axially, within the eye is required. Such a system, as described herein, will prevent vitrectomy beforehand.

### SUMMARY

Provided herein are system, apparatus, method and computer program product embodiments, and combinations and sub-combinations thereof, for a surgical instrument mounted on a surgical robot arm to assist and guide an injection for minimally invasive intraocular surgery.

According to an aspect of the present disclosure, a system for aiding an injection in an intraocular procedure may include a robot arm, a control unit configured to control the robot arm, a targeting instrument mounted on the robot arm, wherein the targeting instrument is configured to aim at a targeted position, wherein an orientation of the robot arm in a direction of the targeted position is saved in a memory to be accessed, and wherein the targeting instrument is further configured to be exchanged with an injection instrument, and the injection instrument mounted on the robot arm, wherein the injection instrument is configured to be positioned at the saved orientation of the robot arm at the targeted position, and wherein the injection instrument is further configured to inject into the targeted position.

In some embodiments, the targeting instrument is a laser device.

In some embodiments, the targeting instrument emits a beam in an orange, green, or red wavelength spectrum at the targeted position.

In some embodiments, the injection instrument is a needle or a subretinal injection needle.

In some embodiments, the targeted position is a surface point on a retina.

In some embodiments, the saved orientation of robot arm at the targeted position includes a representation of a remote center of motion of the robot arm.

In some embodiments, the robot arm is configured to move the injection instrument axially.

According to an aspect of the present disclosure, a method for aiding an injection in an intraocular procedure may include mounting a targeting instrument on a robot arm, positioning the targeting instrument at a targeted position, saving a position of an orientation of the robot arm at the targeted position, exchanging the targeting instrument with an injection instrument on the robot arm, accessing the saved position of the orientation of the robot arm, positioning the injection instrument at the saved position, and injecting at the targeted position via the injection instrument.

According to an aspect of the present disclosure, a method for aiding an injection in an intraocular procedure may include mounting an injection instrument on a robot arm, accessing a saved position, wherein the saved position is an orientation of the robot arm at a targeted position targeted via a targeting instrument previously mounted on the robot arm, positioning the injection instrument at the saved position, wherein the saved position includes a representation of a remote center of motion of the robot arm , and injecting the targeted position via the injection instrument.

### BRIEF DESCRIPTION OF THE FIGURES

The accompanying drawings are incorporated herein and form a part of the specification.
FIG. 1 illustrates a robot including a surgical instrument mounted on a robot arm, according to some embodiments.
FIG. 2A illustrates a procedure for mounting a surgical instrument for aiming at a targeted position, according to some embodiments.
FIG. 2B illustrates a procedure for surgical instrument aiming at a targeted position, according to some embodiments.
FIG. 3A illustrates a procedure for mounting a surgical instrument for injecting at a targeted position, according to some embodiments.
FIG. 3B illustrates a procedure for a surgical instrument injecting at a targeted position, according to some embodiments.
FIG. 4 illustrates a method for a surgical instrument mounted on a surgical robot arm to assist and guide an injection for minimally invasive intraocular surgery, according to some embodiments.
FIG. 5 illustrates an example computer system useful for implementing various embodiments, according to some embodiments.

In the drawings, like reference numbers generally indicate identical or similar elements. Additionally, generally, the left-most digit(s) of a reference number identifies the drawing in which the reference number first appears.

### DETAILED DESCRIPTION

Provided herein are system, apparatus, device, method and computer program product embodiments, and combinations and sub-combinations thereof, for a surgical instrument mounted on a surgical robot arm to assist and guide an injection for minimally invasive intraocular surgery.

In order to provide for a procedure for moving surgical instruments in a direct line, or in an axial direction, different surgical instruments may be used on a robot. Surgical instruments may include, but are not limited to targeting instruments, removal instruments, and injection instruments. By having the injection instruments move axially, a more exact, precise injection at a targeted position may occur. Axial movements allow for the surgical instruments to go through vitreous without exerting traction on the retina. Additionally, such a precise injection provides that vitrectomy is no longer necessary. Avoiding vitrectomy may not only save time as less procedures are required, but also less money on such surgeries is spent. Afterwards, such surgical instruments may be moved laterally.

For example, a surgeon performing such injections manually may have slight movement in their hand during an injection. Even a small movement in any direction may result in an injection at a wrong position within the eye or widening of an induced hole in the retina. Such issues may lead to reflux and inflammation in the eye. Additionally, movement of the surgeon's hand may cause the injection to be too deep in the eye, which may also cause complications. By aiming at a targeted position and saving the targeted position, precision of an injection in the eye may increase. This precision, due to the use of a robot, prevents further complications with the eye later in the patient's life. Although the method and system described herein is refers to intraocular surgery, use of the method and system with a robot may be used for other (micro)surgical surgeries on a human or animal.

The targeted position may be known to the surgeon. The surgeon may have deduced the targeted position from images created from optical coherence tomography (OCT). OCT is an imaging technique often used to images that have a micrometer-level depth resolution by using interferometry with short coherence length light. OCT imaging may use transverse scanning of the light beam and from light reflected from within the eye. Additionally, the targeted position may be known as certain gene therapy medicines should be injected at certain points along the retina. Additionally, the surgeon may use intraoperative optical coherence tomography (iOCT) of the microscope to help identify the targeted position. The surgeon may also know the targeted position during visual feedback from a microscope during the surgery.

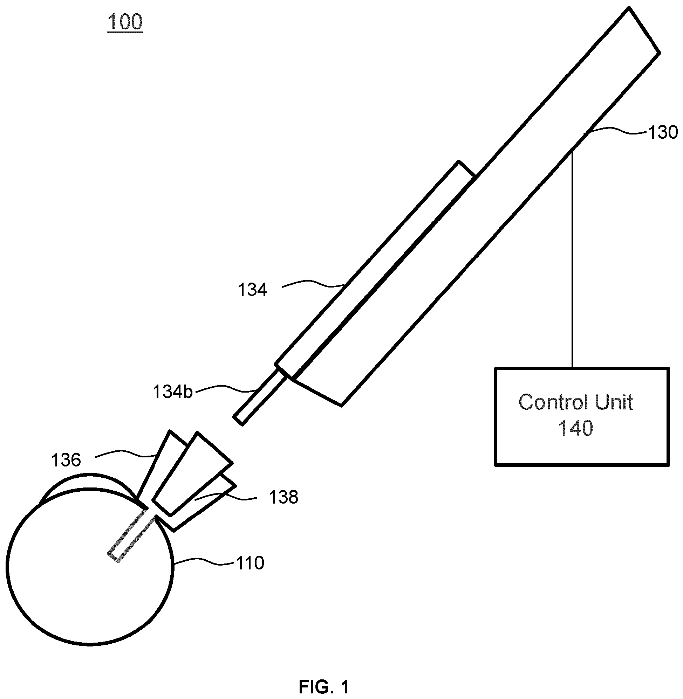

FIG. 1 illustrates a robot 100 including a surgical instrument 134 mounted on a robot arm 130, according to some embodiments. The robot 100 may include the surgical instrument 134 and a distal end of the surgical instrument 134b all mounted on a robot arm 130. The robot arm 130 may be connected to a control unit 140. The surgical instrument 134 may be incident thru a trocar 138 placed in an eye 110 using a trocar port 136 to perform desired functions on the eye 110 during a surgery.

The robot arm 130 may move the surgical instrument 134 in an axial direction and move its orientation. The orientation of the robot arm 130 may be saved for later access.

In some embodiments, the surgical instrument 134 may be a targeting instrument, removal instrument, or an injection instrument, depending on the desired function during the surgery. The trocar 138, often used in minimally invasive eye surgeries, may include a cannula or seal (not pictured). The trocar 138 may assist in withdrawing fluid from the body, such as the eye 110, or may also guide the surgical instrument 134 towards the eye 110 using the trocar port 136. The trocar 138 is the entry point for any surgical instrument 134 to enter the eye 110. The surgical instrument 134 here may be a laser device, microsurgical forceps, needle, micro-pick, or a subretinal injection needle, or the like. For example, the removal instrument may be microsurgical forceps that may assist in removing or peeling membrane from the retina of the eye 110 away.

The robot arm 130 may include a trocar holder (not pictured). The trocar holder may further assist in guiding the surgical instrument 134 towards the eye 110 as the trocar holder ensures a firm connection from the robot arm 130 to the eye 110. The trocar port 136 is inserted on the eye so various functions can be performed on the eye 110. The distal end of the surgical instrument 134b is the part of the surgical instrument 134 that enters the trocar 138 first then heads toward the inside of the eye 110. The distal end of the surgical instrument 134b may assist or aid in targeting within the eye 110, injecting within the eye 110, peeling in the eye 110, scraping debris, such as coagulated proteins chains or membrane, away in the eye 110, or the like.

The trocar 138 may include the trocar port 136 placed on the eye 110 and piercing the surface of the eye 110. The trocar 138 fixes the entry point of the surgical instrument 134. Due to fixing the entry point, the trocar 138 via the trocar holder may act as the remote center of motion (RCM) of the robot arm 130. The RCM may be considered as a remote fixed point, with no physical revolute joint over there, around which the surgical instrument 134 may rotate. The tools, such as the surgical instrument 134, on the robot arm 130 will enter the eye 110 via the trocar 138 and may then be moved laterally (i.e. rotated) around the remote center of motion point and moved axially in the direction of the instrument axis, advancing and retracting the surgical instrument 134 as desired.

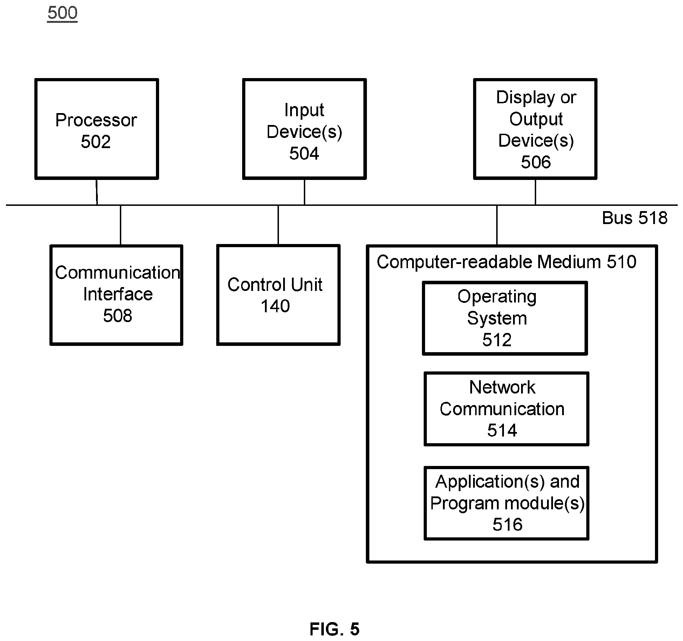

The surgeon may perform a surgery by controlling the robot arm 130 with manipulation of controllers (not pictured) for the robot arm 130 via a control unit 140. The control unit 140 may be included in a computer system 500, further described in FIG. 5. The control unit 140 may assist in controlling the robot 100 or the robot arm 130. The control unit 140 may send an electronic control signal to motors (not pictured) of the robot arm 130. In response to receiving the control signal, the motors may rotate the robot arm 130 into a certain position. Additionally, the motors may move the surgical instrument 134 in an axial position into a certain position.

In some embodiments, the robot arm 130 may move the surgical instrument 134 towards the eye 110 via the trocar 138, the surgical instrument 134 may perform the desired function requested by the surgeon. Typically, the robot arm 130 responds to the surgeon's desired movements. The robot arm 130 has roughly a 1 kHz duty cycle, which may result in a delay of up to 1 ms, which is a minimal delay.

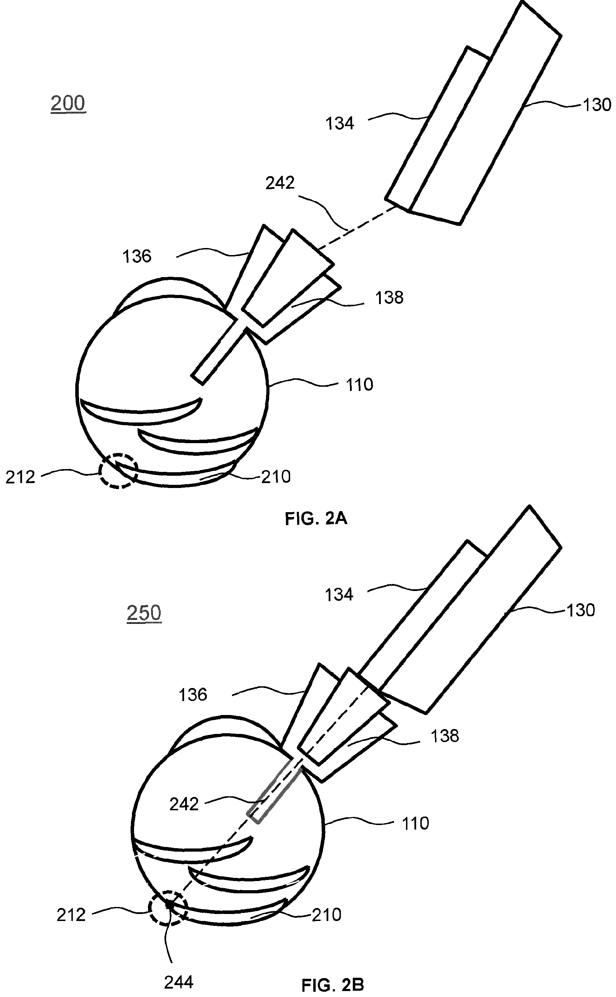

FIG. 2A illustrates a procedure 200 for mounting a surgical instrument 134 on a robot arm 130 for aiming at a targeted position 212, according to some embodiments. The procedure 200 may include the robot arm 130, the surgical instrument 134, a laser beam 242 incident on the eye 110 using a trocar port 136 via the trocar 138. The eye 110 may include material 210. The eye 110 may further include a targeted position 212. The targeted position 212 may be a position where the surgeon would like to inject a substance. While only one targeted position 212 is depicted in FIGS. 2-3, more targeted positions 212 may be envisioned along the eye 110.

The substance to be injected may be a fluid. For example, the fluid may be or comprise a therapeutic agent, which may encompass a wide range of liquid formulations including solutions, suspensions, emulsions, or colloids. The therapeutic agent may contain active pharmaceutical ingredients such as drugs, biological agents including proteins, peptides, nucleic acids, or cells, as well as excipients or additives like stabilizers, preservatives, or buffers. Additionally, the fluid may be designed for various therapeutic purposes such as analgesic, antibiotic, anti-inflammatory, or chemotherapeutic treatments.

Additionally, the targeted position 212 may be a point within the retina of the eye 110, such as the subretinal space, a surface point on the retina, at the surface of an optic disc, or a point within the optic disc of the eye 110. The targeted position 212 may also be a vein, artery, or blood vessel.

The eye 110 may include material 210 throughout the eye, such as coagulated protein chains or membrane, which are difficult for a surgical instrument 134 to move through laterally. The material 210 may, for example, be vitreous or vitreous fibers made from collagen. The material may be a jelly-like substance within the eye 110, appearing like a spaghetti like structure, filling the eye 110. Typically, a change of direction of the surgical instrument 134 would be required as a tip of the surgical instrument 134 would be moved to the targeted position 212, which may result in complications in the eye later.

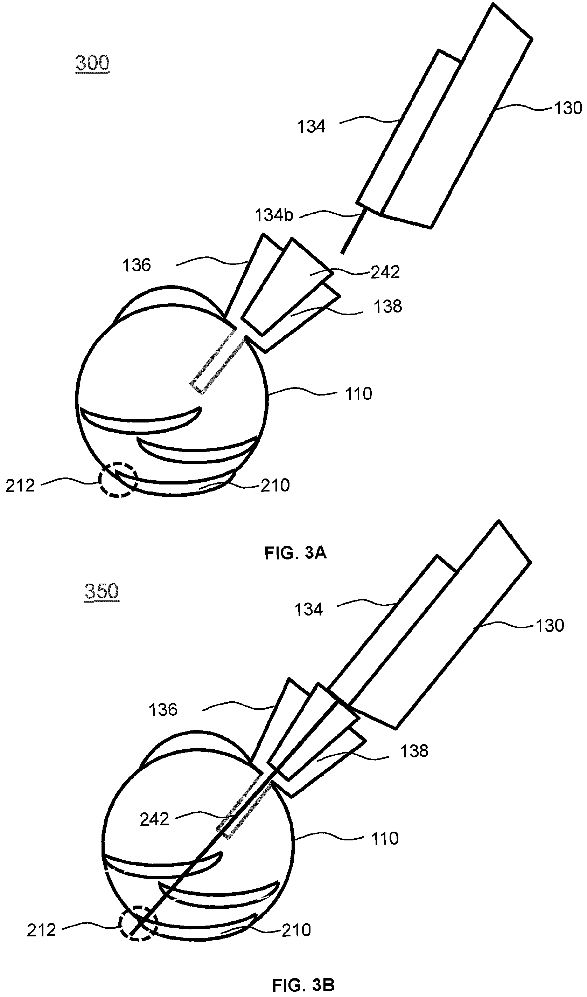

The movement by the surgical instrument 134 thru the material 210 may lead to tension in the retina and possible damage to the retina or eye 110. However, in the present disclosure, the surgical instrument 134 may be a targeting instrument, such as a laser device, and aim at a targeted position 212 directly. By aiming at the targeted position 212, the position of the robot arm 130 aimed at the targeted position 212 may be saved and the laser device does not have to enter the eye 110. Once the position of the robot arm 130 aimed at the targeted position 212 is saved (via the laser dot 244), the surgical instrument 134 may be exchanged for an injection instrument for further procedures, described in FIG. 3. The saved position may include the orientation of the robot arm 130.

In some embodiments, when the surgical instrument 134 may be a targeting instrument, such as a laser device and may be mounted to the robot arm 130. The laser device may be introduced into the eye 110 using the trocar port 136 via the trocar 138. The laser device may assist in looking into the posterior segment of the eye 110, but does not have to enter the eye 110 completely. The posterior segment may include the posterior two thirds of the eye 110 and may further include the vitreous humor, retina, choroid, and optic nerve.

The laser device may be inserted as little as possible through the trocar 138 such that the laser beam properly reaches the targeted location 212 without beam distortion. In some instances, the laser device may be level with the end of the trocar 138, but is not required.

In some embodiments, the laser device may include a laser diode, where a laser beam 242 is emitted from the laser diode and is harmless to the eye 110. The laser beam 242 may be able to get visualization of the robot arm 130 orientation using the macula or near peripheral areas in the retina surrounding the macula, and reflections from the laser beam 242 when emitted in the vitreous matter of the eye 110. The robot arm 130 orientation via the trocar 138 may be recreated using such reflections from the laser beam 242. This orientation will determine the path that the injection instrument will take through the vitreous and into the injection position on the macula or near peripheral areas in the retina surrounding the macula, later described in FIG. 3.

For example, the surgeon may see the retina and laser dot 244 through a microscope (not pictured) and may make adjustments to the orientation of the robot arm 130. The adjustments may be until the laser dot 244 is on the targeted position 212, which is the desired location on the retina by the surgeon. Later, this targeted position 212 is where an injection may occur.

The laser device does not have to be inserted into the trocar 138 in its entirety since only the position of the laser beam 242 generated is required. The laser device enters the eye 110 minimally via the trocar 138.

The robot arm 130 orientation may include rotational around a fixed point due to the remote center of motion of the robot 100. A motor (not pictured) of the robot arm 130 may know such orientation of the robot arm 130 to be saved at the time the surgical instrument 134 is positioned correctly. The saved position specifically includes the orientation of the robot arm 130. The orientation of the robot arm 130 may by defined by two rotational degrees of freedom. The RCM, which is the entry point of the surgical instrument 134 into the trocar 138, stays the same throughout the procedure. The two rotational degrees of freedom may change and be saved such that the targeted position 212 may be accessed again by the surgical instrument 134.

FIG. 2B illustrates a procedure 250 for a surgical instrument 134 aiming at a targeted position 212, according to some embodiments. The procedure 250 may include the robot arm 130, the surgical instrument 134, a laser beam 242 with a laser dot 244 targeted within the eye 110 using the trocar port 136 via the trocar 138, which may include the targeted position 212 near material 210.

Once the surgical instrument 134, here a laser device, has been positioned to aim at the targeted position 212, the surgical instrument 134 may aim the laser beam 242 directly at the targeted position 212. The laser beam 242 may be in an orange, green, or red wavelength spectrum, with a wavelength between 500 to 750 nm. For example, a laser beam 242 of a low intensity may be used, which reduces the chance of harming the eye 110 during the procedure. Once the laser beam 242 is incident on the targeted position 212, a laser dot 244 is created.

In some embodiments, the surgical instrument 134 may target the targeted position 212 after receiving instructions from the robot arm 130. For example, a surgeon may have seen optical scans of the eye 110 of a patient. The surgeon will know where to aim the surgical instrument 134 at the targeted position 212, which is based on the scans of the eye 110 and direct the laser device to the correct position, here the targeted position 212. The surgeon may move the laser device to the targeted position 212 using images seen from a microscope (not pictured) via the controller. Additionally, the surgeon will later know how deep the injection should be, based off the scans of the eye 110. If the injection is too deep within the retina, the eye 110 may be damaged.

The coordinates of the laser device incident on the targeted position 212 may be saved. The targeted position may include an orientation of the robot arm 130 relative to the remote center of motion. The remote center of motion relative to the orientation of the robot arm 130 allows that the robot arm 130 may be brought again back to this exact orientation and pose situation.

Once the orientation of the robot arm 130 position at the targeted position 212 is saved, the targeting instrument, here the laser device, may be retracted from the eye 110. The surgical instrument 134 on the robot arm 130 may be exchanged with a different surgical instrument 134, such as an injection instrument, which may be a subretinal injection needle. The orientation of the robot arm 130 positioned at the targeted position 212 may be saved and may be the desired trajectory of the injection instrument, as described in FIG. 3.

In some embodiments, the surgical instrument may include an integrated light fiber for aiming at the targeted position 212. If an integrated light fiber was utilized, an exchange of instruments would not be required as the injection instrument and light fiber may be integrated together as the surgical instrument 134.

FIG. 3A illustrates a procedure 300 for mounting a surgical instrument 134 for injecting at a targeted position 212, according to some embodiments. The procedure 300 may include the robot arm 130, the surgical instrument 134, the distal end of the surgical instrument 134b to inject in the eye 110 using the trocar port 136 via the trocar 138, which may include the targeted position 212 near material 210.

The robot arm 130 was previously moved slightly away from the eye 110 of the patient, either via the controller on the robot 100 or automatically via a processor, but not decoupled from the eye 110. The robot arm 130 may be moved away such that no lateral movements of the surgical instrument 134 are required to reach the targeted position 212 again. As the location of the RCM point was saved, the control system 140 allows to find the targeted position 212 again. The robot arm 130 is not to be decoupled from the eye 110 via the trocar port 136. The connection between the trocar holder (not pictured) and the trocar 138 may be maintained.

In some embodiments, the orientation of the robot arm 130 may be represented by the motion RCM of the robot arm 130 coupled thru the trocar 138 to the eye 110. That is, the RCM is the point where the surgical instrument 134 may pivot around the trocar 138, without decoupling the robot arm 130 from the trocar 138. Since the orientation of the robot arm 130 was saved due to the movement of the surgical instrument 134 around the RCM, the surgical instrument 134 may access again the saved orientation.

The surgical instrument 134, which was previously the laser device used to target the targeted position 212, may be exchanged for an injection instrument. The injection instrument may be a needle, subretinal injection needle, or the like. Additionally, the surgical instrument 134 may be a micro-pick, forceps, or the like, to perform other desired functions instead of injections during the procedure.

The orientation of the robot arm 130 positioned at the targeted position 212 was previously saved during procedure 250. With the surgical instrument 134 being a subretinal injection needle, it may be ready to be injected directly into the targeted position 212 on the eye 110 via the trocar 138. That is, after exchanging and replacing of the surgical instrument 134, the robot arm 130 may be restored to the orientation of the robot arm 130 positioned at targeted position 212 and have the exact pose according to the saved orientation of the robot arm. Up to now, the surgical instrument 134 may be the subretinal injection needle and will only have to be moved axially towards the eye 110 via the trocar 138, and the material 210, such as coagulated protein chains or membrane, are no longer an issue for the movement of the surgical instrument 134.

FIG. 3B illustrates a procedure 350 for a surgical instrument 134 injecting at a targeted position 212, according to some embodiments. The procedure 350 may include the robot arm 130, the surgical instrument 134, the distal end of the surgical instrument 134b injecting the eye 110 using the trocar port 136 via the trocar 138, which may include the targeted position 212 near material 210.

The surgical instrument 134, such as the injection instrument, more specifically as the subretinal injection cannula, may be inserted into the trocar 138. Before the robot arm 130 moves towards the eye 110 via the trocar 138 again, the robot arm 130 may be brought automatically to the saved targeted position 212. Now that the injection instrument is in the saved orientation of the robot arm 130 position, the distal end of the surgical instrument 134b may move directly towards the targeted position 212. The injection instrument may recall the saved orientation of the robot arm 130 position of the targeted position 212 such that no lateral movement of the injection instrument is required. The surgical instrument 134, here the injection instrument, may then be injected into the targeted position 212 of the eye 110 via the trocar 138 at the distal end of the surgical instrument 134b. Additionally, the depth of the surgical instrument 134 at the targeted position 212 may be adjusted, for example by the surgeon, while the surgical instrument 134 is inserted in an axial direction. The depth may be further controlled using intraoperative optical coherence tomography (iOCT) of the microscope.

In some embodiments, the subretinal injection needle may, for example, inject gene therapy medicine into the eye 110. Gene therapy in a patient's eye is a known treatment that involves replacing non-working genes with healthy copies to slow or halt vision loss. Individuals diagnosed with a genetic eye disease may be a candidate for treatment using gene therapy. The injection described herein may provide such treatment.

In some embodiments, in addition to gene therapy for various diseases in the eye, proteins such as tissue-type plasminogen activator (tPA) may be injected to treat subretinal hemorrhages. tPA may also be injected into retinal veins to dissolve blood clots using such injections described herein. The injection may be at an optic nerve within the eye. The injection may be at a desired depth within the optic nerve. The injections described herein are not limited to injections within the subretinal space.

In some embodiments, aiming at the targeted position 212 with a laser beam 242 first and then moving a surgical instrument 134, such as a subretinal injection needle only in an axial direction may lead to a fast and easy intraocular procedure. The patient will have less complications with the eye 110 later in life.

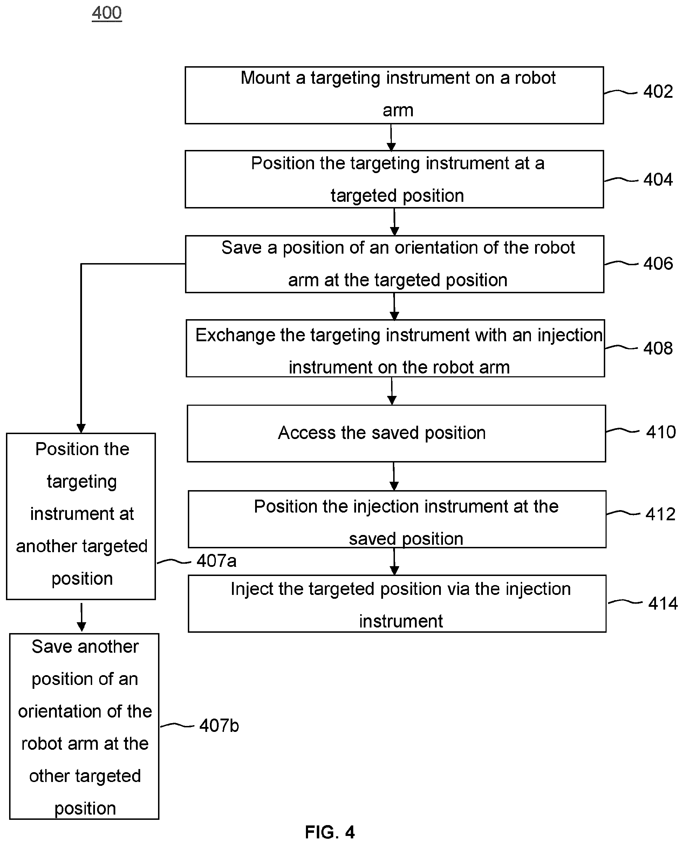

FIG. 4 illustrates a method 400 for a surgical instrument 134 mounted on a surgical robot arm 130 to assist and guide an injection for minimally invasive intraocular surgery, according to some embodiments. Method 400 may be performed by processing logic that may comprise hardware (e.g., circuitry, dedicated logic, programmable logic, microcode, etc.), software (e.g., instructions executing on a processing device), or a combination thereof. It is to be appreciated that not all steps may be needed to perform the disclosure provided herein. Further, some of the steps may be performed simultaneously, or in a different order than shown in FIG. 4, as will be understood by a person of ordinary skill in the art.

Method 400 shall be described with reference to FIGS. 1-3. However, method 400 is not limited to that example embodiment.

In step 402, a targeting instrument may be mounted on a robot arm. The targeting instrument may be a laser device. For example, a targeting instrument such as a laser device may be a surgical instrument 134 and may be mounted on a robot arm 130.

In step 404, the targeting instrument may be positioned at a targeted position. The targeting instrument may emit a beam in an orange, green, or red wavelength spectrum at the targeted position. The targeted position may be a surface point on a retina. Here, the surgical instrument 134 may emit a laser beam 242, that is in the orange, green, or red wavelength spectrum, at a targeted position 212 on a surface point on a retina of the eye 110.

In step 406, a position of an orientation of the robot arm at the targeted position may be saved. A laser dot 244 created by the laser beam 242 indicates the targeted position 212, wherein the corresponding orientation of the robot arm 130 position may be saved and accessed again later.

Alternatively, following step 406, in step 407a, the targeting instrument may be positioned at another targeted position. For example, the surgical instrument 134 may be positioned at another targeted position 212, different from the targeted position of step 404. The other targeted position 212 may be a surface point on a retina. Here, the surgical instrument 134 may emit a laser beam 242, that is in the orange, green, or red wavelength spectrum, at the other targeted position 212 on a surface point on a retina of the eye 110.

Alternatively, following step 407a, in step 407b, another position of an orientation of the robot arm at the other targeted position may be saved. For example, a position of an orientation of the robot arm at the other targeted position 212 may be saved, preferably without overwriting the saved data corresponding to the targeted position 212 acquired in step 406. A laser dot 244 created by the laser beam 242 indicates the other targeted position 212, wherein the corresponding orientation of the robot arm 130 position may be saved and accessed again later. Steps 407a and 407b may also be repeated multiple times to acquire multiple targeted positions 212 throughout the eye 110 and save the corresponding position of an orientation of the robot arm at the corresponding targeted position 212.

In step 408, the targeting instrument may be exchanged with an injection instrument and may be mounted on a robot arm. The injection instrument may be a subretinal injection needle. For example, an injection instrument such as a subretinal injection needle may be surgical instrument 134. The surgical instrument 134 may be mounted on the robot arm 130.

In step 410, a saved position of the orientation of the robot arm may be accessed. The saved position may be the orientation of the robot arm at the targeted position targeted. For example, the saved position may be accessed again such that the saved position is in the direction of the injection instrument, such as surgical instrument 134. The injection instrument may be moved forward in an axial direction towards the targeted position 212.

In step 412, the injection instrument may be positioned at the saved position. The saved position may include the orientation of the robot arm at the targeted position. The positioning of the injection instrument may further include moving the injection instrument axially. For example, the surgical instrument 134, here the subretinal injection needle, may be moved to be positioned at the saved position, which is the targeted position 212. The targeted position 212 may include to the orientation of the robot arm 130. The robot arm 130 may move the surgical instrument 134 axially. The surgical instrument 134 may be moved forward in an axial direction of the surgical instrument 134 until a tip of the surgical instrument, here the subretinal injection needle is placed at the targeted position 212.

In step 414, the targeted position may be injected via the injection instrument. For example, the surgical instrument 134, here the subretinal injection needle, may inject into the targeted position 212. The injection at the targeted position 212 may include a gene therapy injection or the like.

Various embodiments may be implemented, for example, using one or more well-known computer systems, such as computer device 500 shown in FIG. 5. For example, the robot arm 130 may be implemented using combinations or sub-combinations of computer device 500. Also, one or more computer devices 500 may be used, for example, to implement any of the embodiments discussed herein, as well as combinations and sub-combinations thereof.

FIG. 5 shows a computing device 500 for implementing various embodiments, according to some embodiments of the disclosure. For example, computing device 500 may function as system of the robot arm 130 or any portion(s) thereof, or multiple computing devices 500 may function as a system of the robot arm 130.

Computing device 500 may be implemented on any electronic device that runs software applications derived from compiled instructions, including without limitation personal computers, servers, smart phones, media players, electronic tablets, game consoles, email devices, etc. In some implementations, computing device 500 may include one or more processors 502, one or more input devices 504, one or more display devices 506, one or more communication interfaces 508, and one or more computer-readable medium 510. Each of these components may be coupled by bus 518, and in some embodiments, these components may be distributed among multiple physical locations and coupled by a network.

Control unit 140 may assist in controlling the robot 100 or the robot arm 130. The control unit 140 may send an electronic control signal to motors of the robot arm 130. In response to receiving the control signal, the motors rotate the robot arm 130 into a certain position. The surgeon or another user may design a configuration of the control unit 140 by creating the instructions and providing the instructions to the computer device 500. For example, the instructions may be uploaded to a database of the computer device 500 or the surgeon may use input device 504 to control the movement of the robot arm 130.

Display device 506 may be any known display technology, including but not limited to display devices using Liquid Crystal Display (LCD) or Light Emitting Diode (LED) technology. Processor(s) 502 may use any known processor technology, including but not limited to graphics processors and multi-core processors. Input device 504 may be any known input device technology, including but not limited to a keyboard (including a virtual keyboard), mouse, controller, joystick, track ball, and touch-sensitive pad or display.

Bus 518 may be any known internal or external bus technology, including but not limited to ISA, EISA, PCI, PCI Express, NuBus, USB, Serial ATA or FireWire. In some embodiments, some or all devices shown as coupled by bus 518 may not be coupled to one another by a physical bus, but by a network connection, for example. Computer-readable medium 510 may be any medium that participates in providing instructions to processor(s) 502 for execution, including without limitation, non-volatile storage media (e.g., optical disks, magnetic disks, flash drives, etc.), or volatile media (e.g., SDRAM, ROM, etc.).

Computer-readable medium 510 may include various instructions for implementing an operating system 512 (e.g., Mac OS, Windows, Linux). The operating system 512 may be multi-user, multiprocessing, multitasking, multithreading, real-time, and the like. The operating system 512 may perform basic tasks, including but not limited to: recognizing input from input device 504; sending output to display device 506; keeping track of files and directories on computer-readable medium 510; controlling peripheral devices (e.g., disk drives, printers, etc.) which may be controlled directly or through an I/O controller; and managing traffic on bus 518. Network communication 514 may establish and maintain network connections (e.g., software for implementing communication protocols, such as TCP/IP, HTTP, Ethernet, telephony, etc.).

Application(s) and program module(s) 516 may be an application that uses or implements the outcome of processes described herein and/or other processes. For example, application(s) 516 may provide UI and/or UI elements for displaying and/or manipulating updates identified by computer device 500 as described above. In some embodiments, the various processes may also be implemented in operating system 512.

The described features may be implemented in one or more computer programs that may be executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., Objective-C, Java), including compiled or interpreted languages, and it may be deployed in any form, including as a stand-alone program or as a module, component, subroutine, or other unit suitable for use in a computing environment.

Suitable processors 502 for the execution of a program of instructions may include, by way of example, both general and special purpose microprocessors, and the sole processor or one of multiple processors or cores, of any kind of computer. Generally, a processor 502 may receive instructions and data from a read-only memory or a random-access memory or both. The essential elements of a computer may include a processor 502 for executing instructions and one or more memories for storing instructions and data. Generally, a computer may also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data may include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

To provide for interaction with a user, the features may be implemented on a computer having a display device such as an LED or LCD monitor for displaying information to the user and a keyboard and a pointing device such as a mouse or a trackball by which the user may provide input to the computer.

The features may be implemented in a computer system that includes a back-end component, such as a data server, or that includes a middleware component, such as an application server or an Internet server, or that includes a front-end component, such as a client computer having a graphical user interface or an Internet browser, or any combination thereof. The components of the system may be connected by any form or medium of digital data communication such as a communication network. Examples of communication network include, e.g., a telephone network, a LAN, a WAN, and the computers and networks forming the Internet.

The computer system may include clients and servers. A client and server may generally be remote from each other and may typically interact through a network. The relationship of client and server may arise by virtue of computer programs running on the respective computers and having a client-server relationship to each other.

One or more features or steps of the disclosed embodiments may be implemented using an API and/or SDK, in addition to those functions specifically described above as being implemented using an API and/or SDK. An API may define one or more parameters that are passed between a calling application and other software code (e.g., an operating system, library routine, function) that provides a service, that provides data, or that performs an operation or a computation. SDKs may include APIs (or multiple APIs), integrated development environments (IDEs), documentation, libraries, code samples, and other utilities.

The API and/or SDK may be implemented as one or more calls in program code that send or receive one or more parameters through a parameter list or other structure based on a call convention defined in an API and/or SDK specification document. A parameter may be a constant, a key, a data structure, an object, an object class, a variable, a data type, a pointer, an array, a list, or another call. API and/or SDK calls and parameters may be implemented in any programming language. The programming language may define the vocabulary and calling convention that a programmer will employ to access functions supporting the API and/or SDK.

In some implementations, an API and/or SDK call may report to an application the capabilities of a device running the application, such as input capability, output capability, processing capability, power capability, communications capability, etc.

While various embodiments have been described above, it should be understood that they have been presented by way of example and not limitation. It will be apparent to persons skilled in the relevant art(s) that various changes in form and detail may be made therein without departing from the spirit and scope. In fact, after reading the above description, it will be apparent to one skilled in the relevant art(s) how to implement alternative embodiments. For example, other steps may be provided, or steps may be eliminated, from the described flows, and other components may be added to, or removed from, the described systems. Accordingly, other implementations are within the scope of the following claims.

In addition, it should be understood that any figures which highlight the functionality and advantages are presented for example purposes only. The disclosed methodology and system are each sufficiently flexible and configurable such that they may be utilized in ways other than that shown. The breadth and scope of this disclosure should not be limited by any of the above-described exemplary embodiments but should be defined only in accordance with the following claims and their equivalents.

## Claims

1. A system for aiding an injection in an intraocular procedure comprising:
a robot arm;
a control unit configured to control the robot arm;
a targeting instrument mounted on the robot arm, wherein the targeting instrument is configured to aim at a targeted position, wherein an orientation of the robot arm in a direction of the targeted position is saved in a memory of the control unit to be accessed, and wherein the targeting instrument is further configured to be exchanged with an injection instrument; and
the injection instrument mounted on the robot arm, wherein the injection instrument is configured to be positioned at the saved orientation of the robot arm at the targeted position, and wherein the injection instrument is further configured to inject into the targeted position.

2. The system of claim 1, wherein the targeting instrument is a laser device.

3. The system of claims 1 to 2, wherein the targeting instrument emits a beam in an orange, green, or red wavelength spectrum at the targeted position.

4. The system of claims 1 to 3, wherein the injection instrument is a needle or a subretinal injection needle.

5. The system of claims 1 to 4, wherein the targeted position is a surface point on a retina.

6. The system of claims 1 to 5, wherein the saved orientation of the robot arm at the targeted position comprises a representation of a remote center of motion of the robot arm.

7. The system of claims 1 to 6, wherein the robot arm is configured to move the injection instrument axially.

8. A method for positioning a robot arm, the method comprising:
positioning a targeting instrument on the robot arm at a targeted position;
saving an orientation position of an orientation of the robot arm with the targeting instrument positioned at the targeted position;
exchanging the targeting instrument with an injection instrument on the robot arm;
accessing the saved orientation position of the orientation of the robot arm; and
positioning the injection instrument at the saved orientation position.

9. The method of claim 8, further comprising moving the injection instrument in an axial direction until a tip of the injection instrument is placed at the targeted position.

10. The method of claims 8 to 9, wherein the injection instrument is a subretinal injection needle.

11. The method of claims 8 to 10, further comprising:
positioning the targeting instrument on the robot arm at another targeted position; and saving another orientation position of the orientation of the robot arm with the targeting instrument positioned at the other targeted position.

12. The method of claims 8 to 11, wherein the targeted position is a surface point on a retina.

13. The method of claims 8 to 12, wherein the saved orientation of the robot arm at the targeted position comprises a representation of a remote center of motion of the robot arm.
